# EUROPEAN PATENT SPECIFICATION

(11) **EP 4 302 717 B1**
(45) Date of publication and mention of the grant of the patent: **15.07.2026**
(21) Application number: 21929061.6
(22) Date of filing: 04.03.2021
(51) Int. Cl.: A61B 18/18

(54) **MEDICAL TREATMENT TOOL**
MEDIZINISCHES BEHANDLUNGSWERKZEUG
INSTRUMENT DE TRAITEMENT MÉDICAL

(43) Date of publication of application: 10.01.2024
(73) Proprietor: Nikkiso Co., Ltd., Tokyo 150-6022 (JP); Saney Seiko Inc., Asaka-shi, Saitama 351-0021 (JP)
(72) Inventor: KINOSHITA Yoshihiko, Tokyo 150-6022 (JP); OHDAIRA Hisahide, Tokyo 150-6022 (JP); ASANO Takuji, Tokyo 150-6022 (JP); TAKADA Masato, Tokyo 150-6022 (JP); MANPUKU Yasuhiro, Asaka-shi,Saitama 351-0021 (JP); ISHIKAWA Hideki, Asaka-shi,Saitama 351-0021 (JP); ISHIZEKI Yasunori, Asaka-shi,Saitama 351-0021 (JP); OGIWARA Mariho, Asaka-shi,Saitama 351-0021 (JP); MATSUZAKI Masahiro, Asaka-shi,Saitama 351-0021 (JP); KUMAGAI Tomoki, Asaka-shi,Saitama 351-0021 (JP)
(74) Representative: Schmidt, Christian
(86) International application number: PCT/JP2021/008506
(87) International publication number: WO 2022/185488

(56) References cited:
- WO-A1-2010/109908
- WO-A1-2018/147243
- WO-A1-2020/031994
- WO-A1-2020/031994
- WO-A1-2020/256149
- JP-A- 2010 227 555
- JP-A- 2011 135 988
- JP-A- 2020 163 001
- JP-A- 2020 536 636
- US-A1- 2013 190 753
- US-A1- 2020 222 112
- NIKKISO CO., LTD.: "Package Insert Acrossurge Device (Palm S)", PMDA PACKAGE INSERT, PMDA, 31 July 2020 (2020-07-31), pages 1 - 2, XP009539819

## Description

### Technical Field

The present invention relates to a medical treatment instrument which radiates a microwave.

### Background Art

### (Device Using Microwave)

There has been known that a microwave can coagulate (immobilize) biological tissue such as digestive organs, a liver, a bladder, a prostate, a uterus, blood vessels, or intestinal tracts at low temperature. Then, various devices for assisting surgery through use of the microwave have been developed.

### (Related Art)

A large number of medical treatment instruments that radiate a microwave have not only been reported but also been commercially available. For example, the following have been reported.

In Patent Literature 1, there is disclosed "a microwave antenna assembly, comprising: an elongated cooling jacket having proximal and distal ends and an inner lumen defined therebetween; a helical microwave antenna member disposed within at least a portion of the elongated cooling jacket and having an inner conductor and an outer conductor, the inner conductor disposed within the outer conductor, wherein at least a portion of the inner conductor extends distally from the outer conductor and forms a plurality of loops; wherein at least two loops of the plurality of loops form an electrical connection therebetween, and wherein at least a portion of the plurality of loops is configured to deliver microwave energy."

In Patent Literature 2, there is disclosed "an electrosurgical cutting tool having:
a first blade and a second blade, joined at a pivot point, with the first blade comprising a planar body made of a first dielectric material separating a first conductive element on a first surface of the first blade from a second conductive element on a second surface of the first blade, the second surface facing in the opposite direction to the first surface;
actuation means for causing relative rotation between the first blade and the second blade about the pivot point to cause a gap between the first blade and the second blade to change between an open position and a closed position, the relative rotation being substantially in the plane of the blades;
a coaxial transmission line for delivering RF energy and microwave energy to the first blade, the coaxial transmission line having an inner conductor and an outer conductor separated by a second dielectric material,
wherein:
   the inner conductor and the outer conductor are each connected to one of the first conductive element and the second conductive element of the first blade;
   the first conductive element and the second conductive element of the first blade are able to act as an active electrode and a return electrode for sustaining an RF electric field between the first conductive element and the second conductive element, the RF electric field corresponding to RF energy delivered to the first blade by the coaxial transmission line; and
   the first conductive element and the second conductive element of the first blade are able also to act as an antenna structure for radiating a microwave magnetic (EM) field corresponding to microwave energy delivered to the first blade by the coaxial transmission line."

None of the patent literatures discloses or suggests a configuration of a medical instrument according to the present invention.

### Citation List

### Patent Literature

[PTL 1] JP 2008-272472 A
[PTL 2] JP 2019-516426 A

In the context of microwave medical treatments, reference is made also to document US 2020/222112 A1.

### Summary of Invention

### Technical Problem

The related-art medical treatment instrument had one or more of the following various problems. The various problems include, for example, heat generation and radiation caused by microwave radiation, occurrence of sparks, insufficient strength of an insulator, difficulty in cutting a microwave radiation target of antennas, inefficiency of microwave radiation, obstruction of visual field of an operator by antennas, sharpness, occurrence of breakage of a coaxial cable, occurrence of disconnection between a microwave application antenna and a center conductor, difficulty in inserting a distal end of a treatment instrument into fine tissue or a tumor, difficulty in operation by a left-handed operator, low rigidity of a contact portion between antennas and a handle, falling off of an insulator, falling off of antennas from a treatment instrument main body, narrow rotation range of antennas, aging degradation of a microwave radiation switch, and coagulation unevenness. Solution to Problem

The invention and its particularly advantageous embodiments are defined in the appended set of claims.

As a result of extensive studies conducted to solve one or more of the problems described above, the inventors of the present invention has confirmed that the configuration of the medical treatment instrument according to the present invention solves the one or more of the problems described above, and has completed the present invention.

That is, the present invention includes the following aspects.

According to a first aspect, the invention provides a medical treatment instrument, comprising:
a first electrode including a microwave reception antenna;
a second electrode including a microwave application antenna with an insulator, the insulator being installed on a surface opposed to the microwave reception antenna;
a coaxial cable or a semi-rigid cable including a center conductor and an outer conductor, a distal end of the center conductor and a distal end of the outer conductor each being directly or indirectly connected to a terminal end of the microwave application antenna and a terminal end of the microwave reception antenna;
a microwave transmission coaxial cable;
a holder cover, the holder cover accommodating a part of the microwave application antenna and a part of the microwave reception antenna on a terminal end side thereof; and
an inner protrusion of the microwave application antenna, the protrusion extending toward an inner side of the holder cover.

Preferably, a length of the microwave application antenna at a distal end of a blade line in a vertical direction is within a range of 30% to 80% with respect to a length at a terminal end of the blade line in the vertical direction, wherein a length of the microwave reception antenna at a distal end of a peak in the vertical direction is within a range of 30% to 80% with respect to a length at a terminal end of the peak in the vertical direction.

Preferably, an extension of each of the microwave application antenna and the microwave reception antenna from terminal ends to distal ends thereof has a curve with an angle of 10° to 20° with respect to a horizontal extension direction line of the treatment instrument in a state in which the microwave application antenna and the microwave reception antenna are closed, wherein an extension of the microwave reception antenna from the terminal end to the distal end thereof has a curve with an angle of 10° to 40° with respect to the horizontal extension direction line of the treatment instrument in a state in which the microwave application antenna and the microwave reception antenna are opened.

Preferably, an extension of a blade line of the microwave reception antenna from the terminal end to the distal end has a round shape with an angle of 2° to 15° with respect to a horizontal extension of the blade line,
wherein an extension of the peak of the microwave reception antenna from the terminal end to the distal end has a round shape with an angle of 2° to 15° with respect to a horizontal extension of the peak, and
wherein an extension of the peak of the microwave application antenna from the terminal end to the distal end has a round shape with an angle of 2° to 15° with respect to the horizontal extension of the peak.

According to a further aspect, the microwave reception antenna has an edge shape, wherein the edge shape is represented as "thickness × 0.2 to 1.5".

According to a further aspect, the instrument comprises an antenna cover, wherein the holder is accommodated in the holder cover, and wherein the antenna cover accommodates a connection portion between the microwave application antenna and the coaxial cable, or the semi-rigid cable.

Preferably, the antenna cover includes a straight portion that accommodates a connection portion between the microwave application antenna and the coaxial cable.

Preferably, the medical treatment instrument includes a handle 1, a handle 2, an arm 1, an arm 2, a finger hook 1, a finger hook 2, a ring 1, and a ring 2 in order to form forceps, wherein the finger hook 1 and the ring 1 are each located in a substantially symmetrical manner with the finger hook 2 and the ring 2 with respect to a center line of the treatment instrument.

Preferably, the holder cover includes a coaxial cable protection member on a terminal end side of the cover, wherein the coaxial cable protection member is installed between the handle 1 and the handle 2, and the coaxial cable.

In an embodiment, the medical treatment instrument includes a main support plate and an auxiliary support plate, wherein the main support plate and the auxiliary support plate are connected to a terminal end of the microwave reception antenna, and the terminal end of the microwave reception antenna and a distal end of the auxiliary support plate are connected to each other with a distal end of the main support plate sandwiched therebetween.

Preferably, the handle 2 has a distal end shape that prevents tissue from entering a gap defined between the holder cover and the handle 2.

Preferably, a distal end of the insulator has a recessed shape or a protruding shape, and the distal end of the microwave application antenna has a protruding shape or a recessed shape.

In an embodiment, a distal end of the semi-rigid cable has a protruding shape, and the terminal end of the microwave application antenna has a connection groove that is a slit formed in a recessed manner, wherein the protrusion and the connection groove form a connection structure.

In an embodiment, the medical treatment instrument includes a protrusion that penetrates through the holder and extends toward an inner side of the insulator, and the insulator includes a protrusion accommodation groove for accommodating the protrusion, wherein the protrusion and the protrusion accommodation groove form the connection structure.

In an embodiment, the medical treatment instrument includes a protrusion that penetrates through the holder and extends toward an upper side of the insulator, and the insulator includes a protrusion accommodation groove for accommodating the protrusion, wherein the protrusion and the protrusion accommodation groove form the connection structure.

In an embodiment, the medical treatment instrument includes a traction rod, and the traction rod includes a pin that extends in an inner direction of the treatment instrument at an end of the rod, wherein the microwave reception antenna includes a hole at a peak-side terminal end of the antenna, and the pin is inserted into the hole.

In an embodiment, the medical treatment instrument includes a main body cover including any one or more of a shaft, a rotator, a hand switch, a lever, and a grip portion.

Preferably, the medical treatment instrument includes the rotator, a shaft that penetrates inside of the rotator, the main body cover, and a semi-rigid cable rotation connector and a coaxial cable rotation connector that are accommodated in the main body cover, wherein the semi-rigid cable and the coaxial cable are each connected to each other through intermediation of the semi-rigid cable rotation connector and the coaxial cable rotation connector.

Alternatively, the medical treatment instrument includes a hand switch, the main body cover, a microwave radiation substrate that receives power of a hand switch accommodated in the main body cover, and a switch cover accommodated in the main body cover, and
wherein the switch cover is located between the hand switch and the microwave radiation substrate.

Preferably, a shape of the holder cover on a terminal end side of the cover is a tapered shape.

In an embodiment, the shaft includes one or more or a plurality of claw-shaped portions, and the holder cover includes one or more or a plurality of through holes that accommodate the claw-shaped portions, wherein the claw-shaped portions are inserted into the through holes.

### Advantageous Effects of Invention

The medical treatment instrument according to the present invention has any one or more of the following effects.
(1) Suppression of heat generation and radiation by a microwave radiation.
(2) Improvement in strength of an insulator.
(3) Easy insertion into a microwave radiation target tissue of antennas.
(4) Improvement in cutting of a microwave radiation target tissue of antennas.
(5) Efficient microwave radiation.
(6) Improvement in visual field of an operator.
(7) Improvement in sharpness for a microwave radiation target tissue of antennas.
(8) Easy insertion of a distal end of a treatment instrument into fine tissue or a tumor.
(9) No coagulation unevenness.
(10) Suppression of occurrence of sparks.

A medical treatment instrument (100) including a handle according to the present invention further has any one or more of the following effects in addition to any one or more of the items (1) to (10) described above.
(11) Prevention effect for breakage of a coaxial cable.
(12) Easy operation regardless of whether an operator is right-handed or left-handed.
(13) Improvement in rigidity of a contact portion between antennas and a handle and rigidity of the handle.

A medical treatment instrument (200) including a handle according to the present invention further has any one or more of the following effects in addition to any one or more of the items (1) to (10) described above.
(14) Prevention for falling off of an insulator from a microwave application antenna.
(15) Prevention effect for disconnection between a microwave application antenna and a center conductor.
(16) Prevention of coming off of antennas from a treatment instrument main body while maintaining strength of two antennas.
(17) Antennas can be rotated 360°.
(18) Aging degradation prevention for a microwave radiation switch.
(19) Prevention for falling off of a holder cover.

### Brief Description of Drawings

FIG. 1 is an overall view of a medical treatment instrument (100) including a handle.
FIG. 2 is an overall view of a medical treatment instrument (200) including a lever.
FIG. 3 is an explanatory view for illustrating an inner protrusion (20) of a microwave application antenna of the medical treatment instrument (100).
FIG. 4 is an explanatory view for illustrating an inner protrusion (20) of a microwave application antenna of the medical treatment instrument (200).
FIG. 5 is a view for illustrating shapes of antennas of the medical treatment instrument (100) (an upper view is a view for illustrating a state in which the antennas are opened, and a lower view is a view for illustrating a state in which the antennas are closed).
FIG. 6 is a view for illustrating shapes of antennas of the medical treatment instrument (200) (an upper view is a view for illustrating a state in which the antennas are opened, and a lower view is a view for illustrating a state in which the antennas are closed).
FIG. 7 is a view for illustrating round shapes of the antennas of the medical treatment instrument (100).
FIG. 8 is a view for illustrating round shapes of the antennas of the medical treatment instrument (200).
FIG. 9 is a sectional view of the medical treatment instrument (100) for illustrating a coaxial cable (17), a holder cover (19), a holder (22), an antenna cover (114), and a straight portion (101) of the antenna cover.
FIG. 10 is a sectional view of the medical treatment instrument (200) for illustrating a holder cover (19), a holder (22), and a semi-rigid cable (206).
FIG. 11 is an explanatory view for illustrating a curved shape of the antenna of the medical treatment instrument (100).
FIG. 12 is an explanatory view for illustrating a curved shape of the antenna of the medical treatment instrument (200).
FIG. 13 is an explanatory view for illustrating an edge shape of a microwave reception antenna (10).
FIG. 14 is an explanatory view for illustrating a symmetrical structure of the medical treatment instrument (100).
FIG. 15 is an explanatory view for illustrating a coaxial cable protection member (110) of the medical treatment instrument (100).
FIG. 16 is an explanatory view for illustrating a contact portion between the holder cover and the handle of the medical treatment instrument (100) (an upper view is a view for illustrating a state in which the antennas are opened, and a lower view is a view for illustrating a state in which the antennas are closed).
FIG. 17 is an explanatory view for illustrating structure of a microwave application antenna (12) including an insulator (14) of the medical treatment instrument (200).
FIG. 18 is an explanatory view for illustrating structure of the semi-rigid cable (206) of the medical treatment instrument (200).
FIG. 19 is an explanatory view for illustrating fixation between the microwave reception antenna (10) and the microwave application antenna (12) in the medical treatment instrument (200).
FIG. 20 is an explanatory view for illustrating an opening and closing operation of the microwave reception antenna (10) in the medical treatment instrument (200).
FIG. 21 is an explanatory view for illustrating 360° rotation of the antenna in the medical treatment instrument (200).
FIG. 22 is an explanatory view for illustrating a switch cover (215) of the medical treatment instrument (200).
FIG. 23 is an explanatory view for illustrating structure of a terminal end of the microwave reception antenna (10) of the medical treatment instrument (100).
FIG. 24 is an enlarged view for illustrating the structure of the terminal end of the microwave reception antenna (10) of the medical treatment instrument (100).
FIGS. 25A and B show coagulation results obtained using the medical treatment instrument (100).

Hereinafter, the present invention is described with reference to the drawings. However, the present invention is not limited to medical treatment instrument illustrated in the drawings.

### (Medical Treatment Instrument)

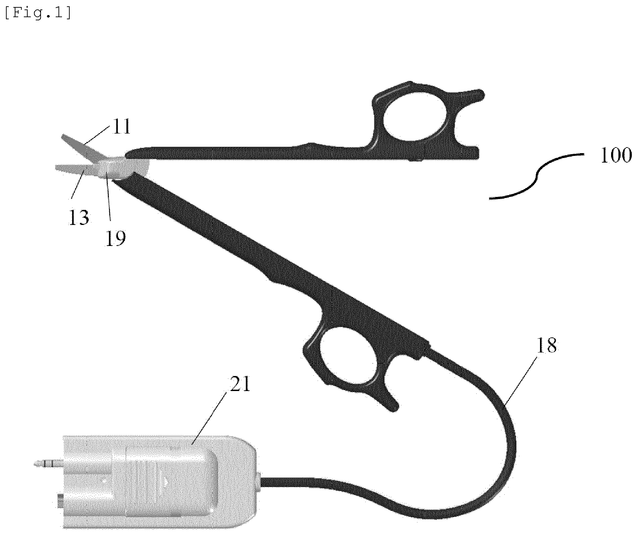

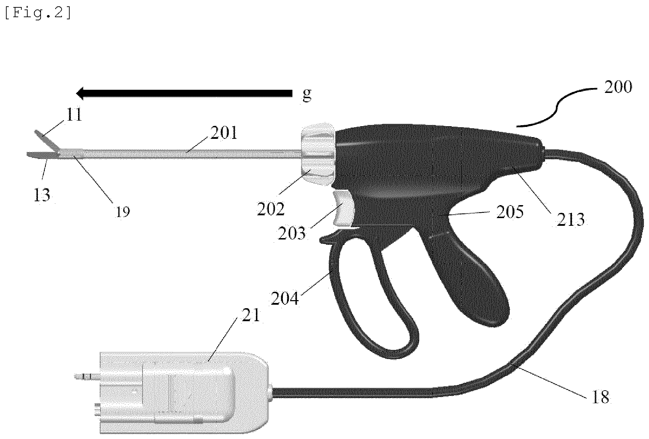

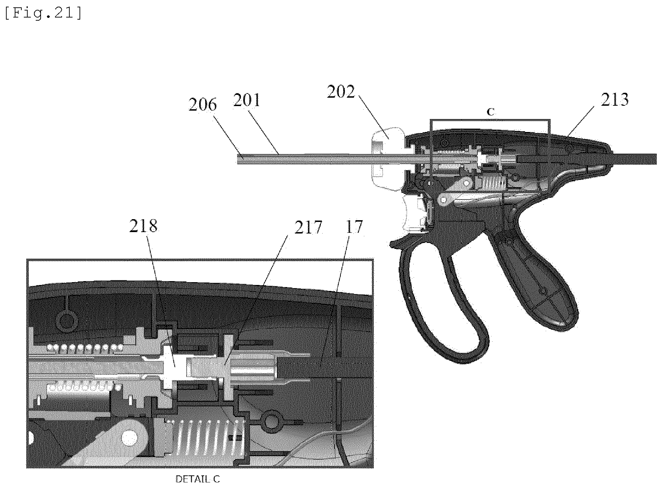

A medical treatment instrument (1) according to the present invention relates to a microwave radiation instrument (in particular, microwave radiation surgical instrument) capable of irradiating tissue with a microwave (see FIG. 1 to FIG. 24), and mainly includes the following two configurations of the medical treatment instruments.
A: A medical treatment instrument (100) including a handle (see FIG. 1).
B: A medical treatment instrument (200) including a lever (see FIG. 2).

### (Configuration common to Medical Treatment Instruments (100) (200))

The medical treatment instrument (1) according to the present invention includes at least the following configuration.

A first electrode (11) including a microwave reception antenna (10).

A second electrode (13) including a microwave application antenna (12) with an insulator (14). The insulator (14) is installed on a surface opposed to the microwave reception antenna (12).

A coaxial cable (17) or a semi-rigid cable (206) including a center conductor and an outer conductor.

A distal end of the center conductor and a distal end of the outer conductor are each directly or indirectly connected to a terminal end of the microwave application antenna (12) and a terminal end of the microwave reception antenna (10).

A microwave transmission coaxial cable (18).

A holder cover (19). The holder cover (19) accommodates a part of the microwave application antenna (12) and a part of the microwave reception antenna (10) on a terminal end side thereof.

An inner protrusion (20) of the microwave application antenna. The inner protrusion (20) of the microwave application antenna extends toward an inner side of the holder cover.

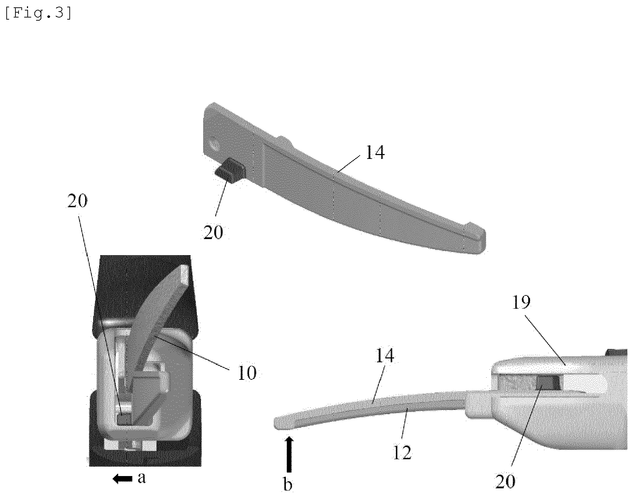

In addition, a distal end direction of the medical treatment instrument (1) according to the present invention corresponds to an extension direction of an arrow (arrow "g") illustrated in FIG. 2 and FIG. 3. Further, a terminal end side of an electrode corresponds to a holder cover side.

The microwave transmission coaxial cable (18) is directly or indirectly coupled to a connector (21). A microwave is supplied to the medical treatment instrument (1) via the connector (21).

### (Holder Cover)

With regard to the medical treatment instruments (100) (200) according to the present invention, the holder cover (19) prevents heat generation and radiation caused by a microwave radiation (see FIG. 1 and FIG. 2). Accordingly, the medical treatment instruments (100) (200) according to the present invention can prevent heat damage of living tissue that is not a microwave radiation target.

Although the material of the holder cover (19) is not particularly limited as long as the heat generation and radiation caused by the microwave radiation can be prevented, there can be exemplified, for example, a resin material, in particular, a polyether ether ketone resin and the like.

### (Inner Protrusion of Microwave Application Antenna)

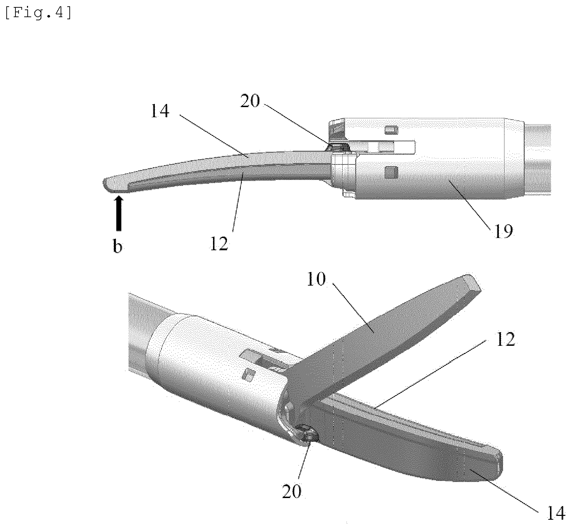

With regard to the medical treatment instruments (100) (200) according to the present invention, the inner protrusion (20) of the microwave application antenna extends toward, as illustrated in FIG. 3 and FIG. 4, an inner side (arrow "a") of a holder (22) accommodated in the holder cover (19). Although the material of the inner protrusion (20) of the microwave application antenna is not particularly limited, there can be exemplified ceramic, a resin material (in particular, a polyether ether ketone resin), stainless steel, and the like.

When load is applied in a load direction (arrow "b"), a distal end of the inner protrusion (20) of the microwave application antenna is brought into contact with an inner side of the holder (22) and then the load is applied with the protrusion serving as a fulcrum. Thus, strength of the insulator (14) can be improved.

### (Overall Shape of Antenna)

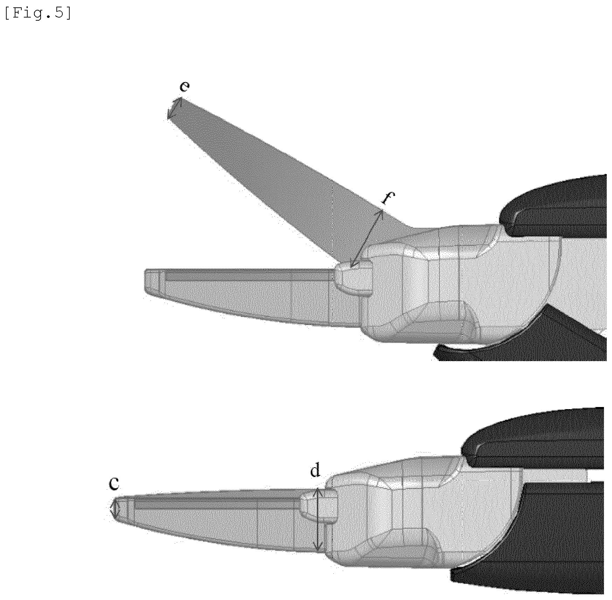

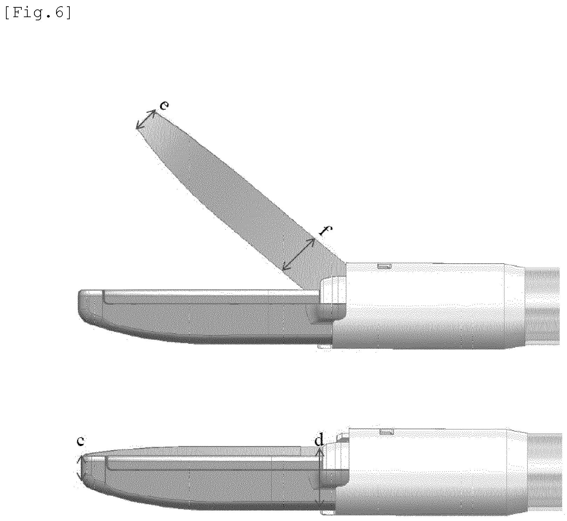

With regard to the medical treatment instruments (100) (200) according to the present invention, it is preferred that a length (arrow "c") of the microwave application antenna (12) at a distal end of a blade line in a vertical direction be within a range of 30% to 80% with respect to a length (arrow "d") at a terminal end of the blade line in the vertical direction (see FIG. 5 and FIG. 6).

It is preferred that a length (arrow "e") of the microwave reception antenna (10) at a distal end of a peak in the vertical direction be within a range of 30% to 80% with respect to a length (arrow "f") at a terminal end of the peak in the vertical direction.

As described above, the overall shapes of both antennas become sharper toward the distal ends thereof, thus at the time of microwave heat and separation of adhesion of living tissue, insertion into tissue becomes easier.

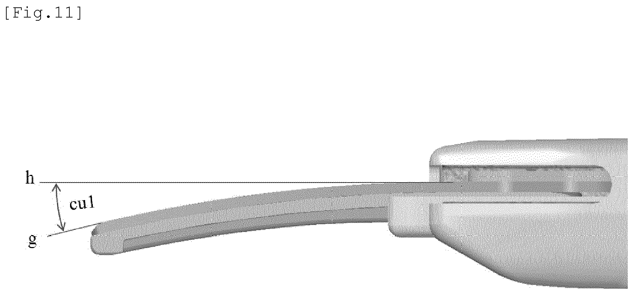

With regard to the medical treatment instrument (100) according to the present invention, an extension (see line "g") of each of the microwave application antenna (12) and the microwave reception antenna (10) from terminal ends to distal ends thereof has a curve (cu1) with an angle of 10° to 20° (see FIG. 11) with respect to a horizontal extension direction line (see line "h") of the treatment instrument in a state in which the microwave application antenna (12) and the microwave reception antenna (10) are closed. In addition, an extension of the microwave reception antenna (10) from the terminal end to the distal end thereof has a curve with an angle of 10° to 40° with respect to the horizontal extension direction line of the treatment instrument in a state in which the microwave application antenna (12) and the microwave reception antenna (10) are opened.

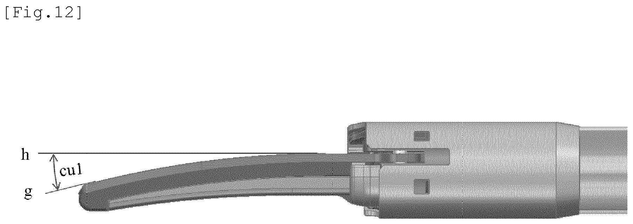

With regard to the medical treatment instrument (200) according to the present invention, an extension (see line "g") of each of the microwave application antenna (12) and the microwave reception antenna (10) from terminal ends to distal ends thereof has a curve (cu1) with an angle of 10° to 20° (see FIG. 12) with respect to a horizontal extension direction line (see line "h") of the treatment instrument in a state in which the microwave application antenna (12) and the microwave reception antenna (10) are closed. In addition, an extension of the microwave reception antenna (10) from the terminal end to the distal end thereof has a curve with an angle of 10° to 40° with respect to the horizontal extension direction line of the treatment instrument in a state in which the microwave application antenna (12) and the microwave reception antenna (10) are opened.

With the curve described above, there is provided an effect that both antennas allow visual field of an operator (visual field of a microwave radiation target tissue) to see more easily.

Further, when a range of the curve is different between the opened state described above and a closed state (curve of the microwave reception antenna is made sharper than the curve of the microwave application antenna), at the time of closing the microwave reception antenna, the microwave application antenna (12) including the insulator (14) and the microwave reception antenna function as scissors by gradually deforming the curve of the microwave application antenna so as to extend along the microwave application antenna.

### (Round Shape of Antenna)

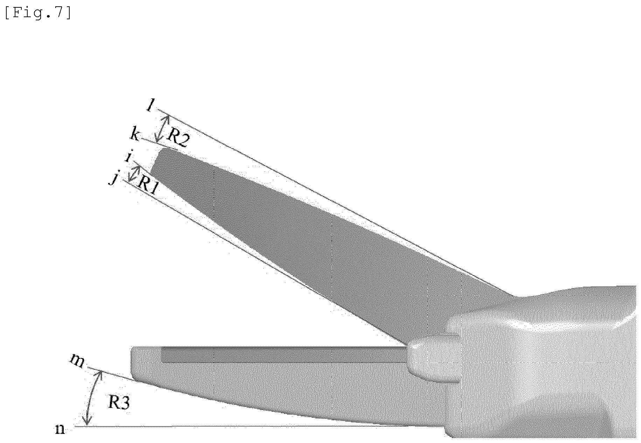

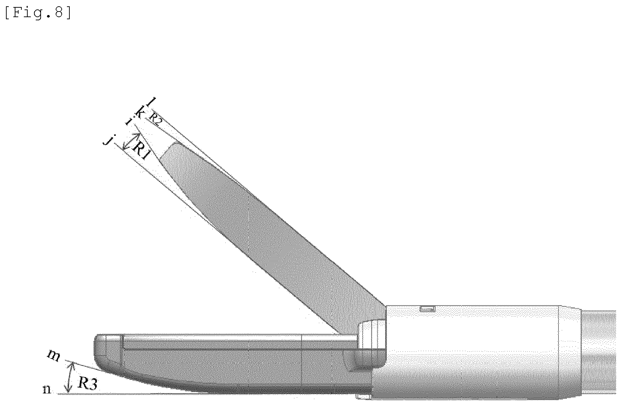

With regard to the medical treatment instruments (100) (200) according to the present invention, an extension (line "i") of the microwave reception antenna (10) from a terminal end to a distal end of a blade line has a round shape (R1) with an angle of 2° to 15° with respect to a horizontal extension (line "j") of the blade line (see FIG. 7 and FIG. 8). This R1 shape has an effect of improving "cutting in a crushing manner".

Similarly, an extension (line "k") of a peak of the microwave reception antenna (10) from a terminal end to a distal end has a round shape (R2) with an angle of 2° to 15° with respect to a horizontal extension (line "1") of the peak. Similarly, an extension (line "m") of the peak of the microwave application antenna (12) from a terminal end to a distal end has a round shape (R3) with an angle of 2° to 15° with respect to a horizontal extension (line "n") of the peak.

Those R2 and R3 shapes have an effect of "smoothly inserting into the microwave radiation target tissue without damaging the tissue".

The antennas having those R1, R2, and R3 shapes are absent in the cutting in a crushing manner, compared to the antennas without a round shape. Further, the antennas can be smoothly inserted into the microwave radiation target tissue without damaging the tissue.

That is, the round shape of the microwave reception antenna has a cutting improvement effect for the microwave radiation target tissue.

### (Edge Shape of Microwave Reception Antenna)

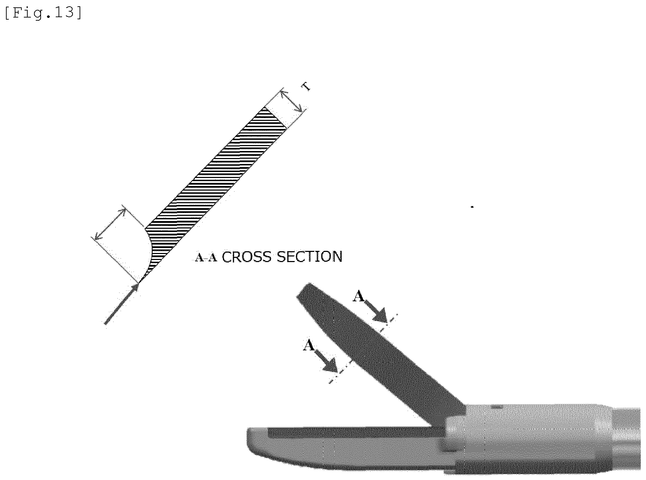

With regard to the medical treatment instruments (100) (200) according to the present invention, the microwave reception antenna (10) has an edge shape as illustrated in FIG. 13. The reference symbol "T" indicates an edge shape and is within a range of "thickness (T) × 0.2 to 1.5".

The edge shape of the microwave reception antenna (10) improves sharpness compared to the antennas without the edge shape as illustrated in FIG. 13.

### (Holder for Connection Portion between Microwave Application Antenna and Coaxial Cable or Semi-rigid Cable)

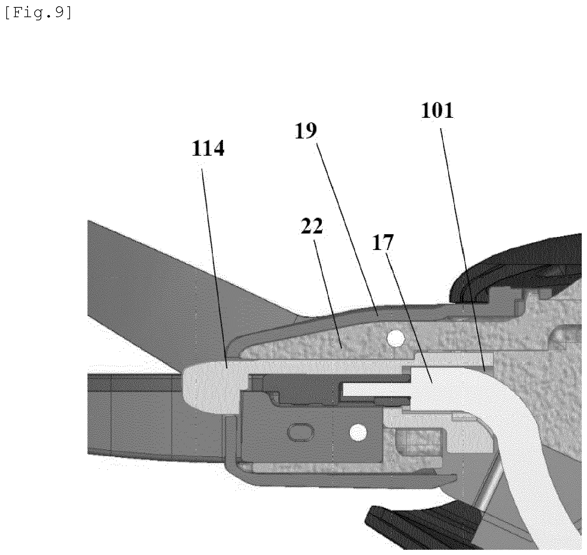

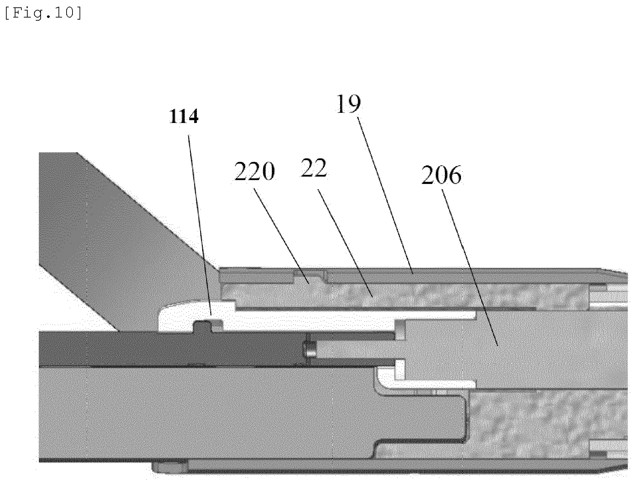

With regard to the medical treatment instruments (100) (200) according to the present invention, as illustrated in FIG. 9 and FIG. 10, the holder cover (19) accommodates the holder (22).

Although the material of the holder (22) is not particularly limited, a stainless steel material can be exemplified. The holder (22) can prevent a microwave from being transmitted to parts other than the microwave reception antenna (10). Accordingly, the holder (22) can efficiently transmit a microwave to the microwave reception antenna (10).

The antenna cover (114) accommodates a connection portion between the microwave application antenna and the coaxial cable.

With regard to the medical treatment instrument (100) according to the present invention, wherein the antenna cover (114) includes a straight portion (101) that accommodates the connection portion between the microwave application antenna and the coaxial cable. The straight portion (101) is a hollow structure (hollow tube) that can accommodate the connection portion from a terminal end side and has a certain axial length (for example, 5 mm) so that the microwave application antenna can substantially connect to (fit to) the coaxial cable in a straight manner, thus buckling of the center conductor of the coaxial cable can be prevented so that breakage due to the buckling does not occur. In addition, although the material of the antenna cover (114) is not particularly limited, a resin of the insulator (in particular, PEEK resin) can be exemplified.

### (Structure of Medical Treatment Instrument (100))

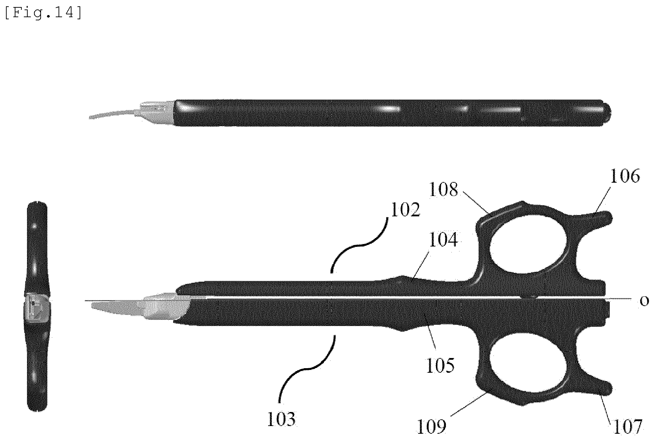

The medical treatment instrument (100) according to the present invention further includes, as illustrated in FIG. 14, as required, a handle 1 (102), a handle 2 (103), an arm 1 (104), an arm 2 (105), a finger hook 1 (106), a finger hook 2 (107), a ring 1 (108), and a ring 2 (109).

The finger hook 1 (106) and the ring 1 (108) are each located in a substantially symmetrical manner with the finger hook 2 (107) and the ring 2 (109) with respect to a center line (line "o") of the treatment instrument. Accordingly, regardless of whether an operator is right-handed or left-handed, the operator can easily operate the medical treatment instrument (100) according to the present invention.

Although the materials of the handle, the arm, the finger hook, and the ring are not particularly limited, polycarbonate (in particular, polycarbonate with glass fiber) can be exemplified.

### (Coaxial Cable Protection Member of Medical Treatment Instrument (100))

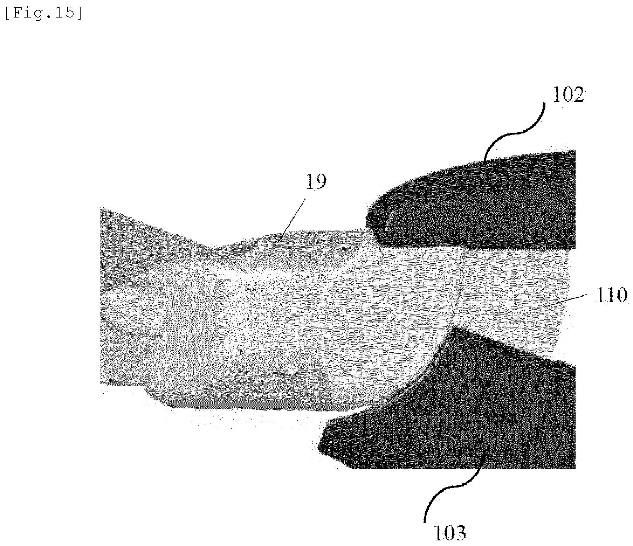

The medical treatment instrument (100) according to the present invention includes, as illustrated in FIG. 15, a coaxial cable protection member (110) on the terminal end side of the holder cover (19). In order to prevent the handle 1 (102) and the handle 2 (103) from contacting with the coaxial cable (17), the coaxial cable protection member (110) is installed between the handle 1 (102) and the handle 2 (103), and the coaxial cable (17). With the coaxial cable protection member (110) installed in that position, the coaxial cable (17) is protected. Further, by overlapping the coaxial cable protection member (110) with the handle 1 (102) and the handle 2 (103), the overall rigidity of the medical treatment instrument (100) (in particular, the rigidity of a contact portion between the holder cover (19) or the antennas and the handle 1 (102) and the handle 2 (103)) can be improved.

Although the material of the protection member is not particularly limited as long as the heat generation and radiation caused by the microwave radiation can be prevented, there can be exemplified, for example, a resin material, in particular, a polyether ether ketone resin and the like.

### (Structure of Microwave Reception Antenna (10) of Medical Treatment Instrument (100))

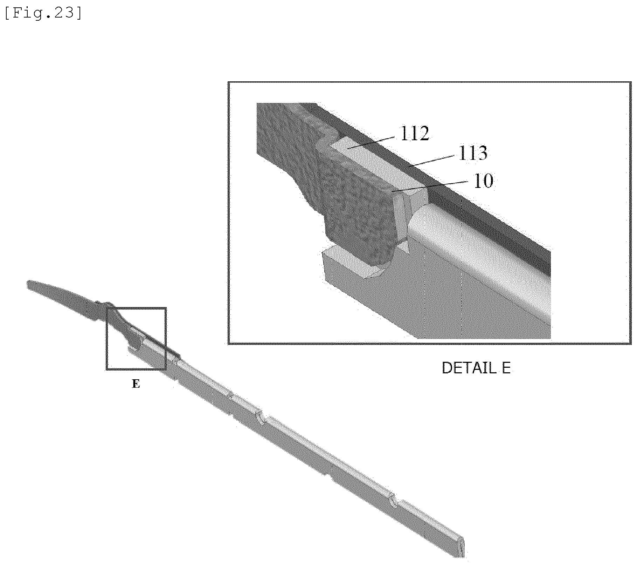

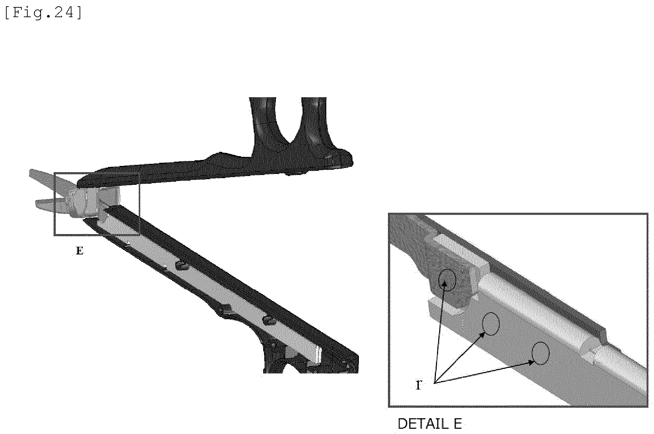

The terminal end of the microwave reception antenna (10) of the medical treatment instrument (100) according to the present invention is preferably, as illustrated in FIG. 23, connected to a main support plate (112) and an auxiliary support plate (113). Further, the main support plate (112) may be formed of a plurality of plates by hemming bending and the like. More specifically, the terminal end of the microwave reception antenna (10) and a distal end of the auxiliary support plate (113) are connected to each other with a distal end of the main support plate (112) sandwiched therebetween. As an example of the connection method, as illustrated in FIG. 24, a plurality of regions ("r") are subjected to a spot welding.

Connecting the terminal end of the microwave reception antenna (10) to the distal ends of the main support plate (112) and the auxiliary support plate (113) can improve the rigidity of a contact portion between the antennas and the handle and the rigidity of the handle.

Although the materials of the main support plate and the auxiliary support plate are not particularly limited, stainless steel (SUS 304) can be exemplified.

### (Contact Portion between Holder Cover and Handle in Medical Treatment Instrument (100))

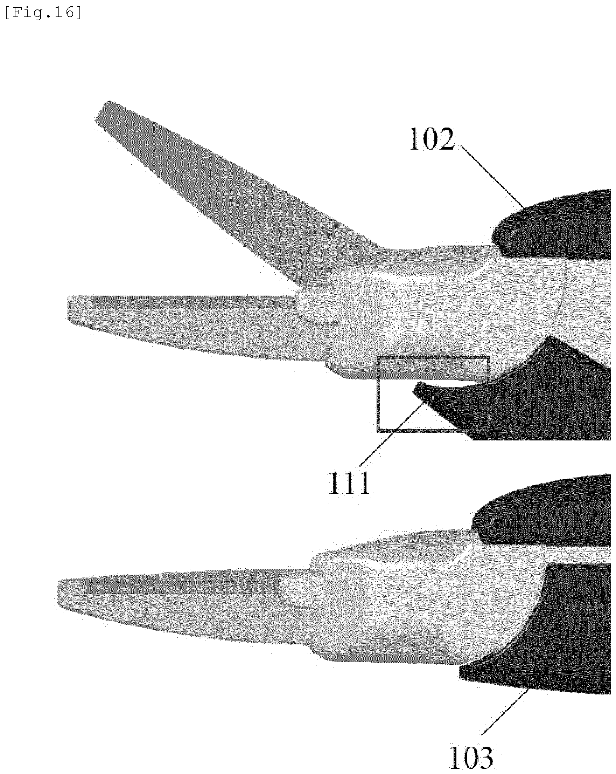

With regard to the medical treatment instrument (100) according to the present invention, as illustrated in FIG. 16, through operation of the handle 1 (102) and/or the handle 2 (103), even when a distal end portion of the handle 2 (103) is moved, the handle 2 (103) has a distal end shape (111) that prevents tissue from entering a gap defined between the cover and the distal end portion of the handle 2 (103).

### (Structure of Medical Treatment Instrument (200))

The medical treatment instrument (200) according to the present invention further includes, as illustrated in FIG. 2, as required, a main body cover (213) including a shaft (201), a rotator (202), a hand switch (203), a lever (204), and a grip portion (205). The lever (204) is mounted in a feely swingable manner, and the movement of the swinging is converted into back and forth movement of a traction rod (207) including a pin to be described later by a publicly known slider crank mechanism.

Further, the rotator (202) is, as described later, a mechanism that can be rotated 360° with the shaft (201).

### (Structure of Microwave Application Antenna (12) including Insulator (14) of Medical Treatment Instrument (200))

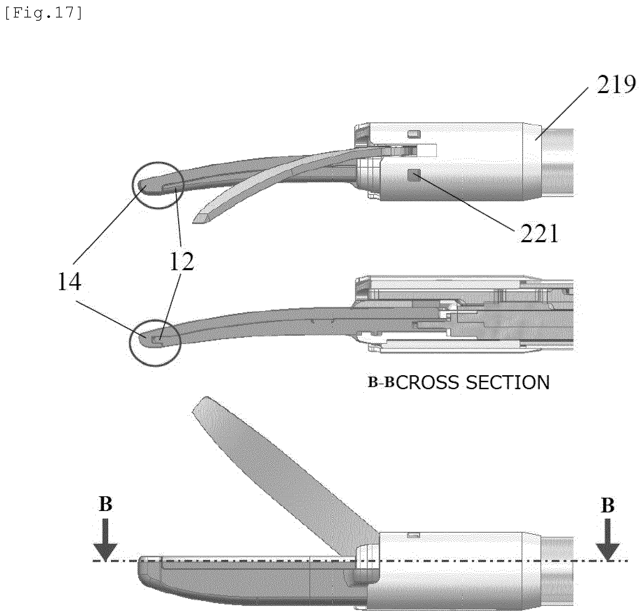

With regard to the medical treatment instrument (200), as illustrated in FIG. 17, it is preferred that a distal end of the insulator (14) have a recessed shape or a protruding shape, and the distal end of the microwave application antenna (12) has a protruding shape or a recessed shape. The insulator (14) and the microwave application antenna (12) are connected to each other by the protrusion and the recess. Accordingly, the insulator (14) suppresses falling off from the microwave application antenna (12).

### (Structure of Semi-rigid Cable (206) of Medical Treatment Instrument (200))

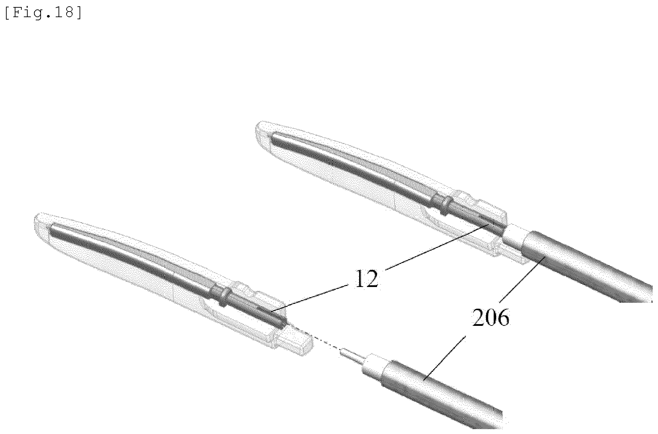

In the medical treatment instrument (200) according to the present invention, it is preferred that, as illustrated in FIG. 18, the semi-rigid cable (206) have a protruding shape at a distal end thereof (center conductor) and the microwave application antenna (12) have a semi-rigid cable connection groove including a slit formed in a recessed manner at a terminal end thereof. With the connection structure (fitting structure, connecting structure) formed by the protrusion and the connection groove, the connection between the microwave application antenna (12) and the center conductor can be stably connected with each other, thus disconnection (release of connection) between the semi-rigid cable (206) and the microwave application antenna (12) can be prevented.

### (Fixation between Microwave Reception Antenna (10) and Microwave Application Antenna (12) in Medical Treatment Instrument (200))

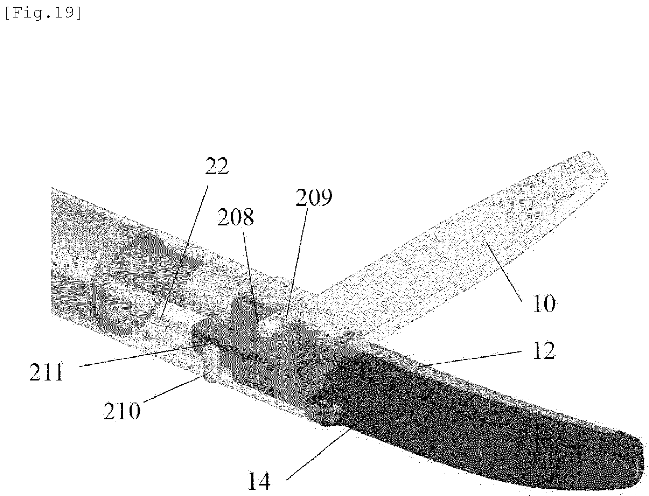

As illustrated in FIG. 19, the medical treatment instrument (200) according to the present invention includes "protrusion (208) that extends toward an inner surface of the insulator (14)" that is a protrusion (for example, pin) that penetrates through the holder (22) and extends toward an inner direction of the insulator (14). The insulator (14) includes "protrusion accommodation groove (209) located on the inner surface of the insulator (14)" which is a protrusion accommodation groove (for example, recessed shape) for accommodating the protrusion. When the protrusion is accommodated into the accommodation groove, the strength of the microwave reception antenna can be maintained by not forming a hole shape in the microwave reception antenna (10).

Further, as illustrated in FIG. 19, the medical treatment instrument (200) according to the present invention includes "protrusion (210) that extends toward the insulator (14)" which is a protrusion (for example, pin) that penetrates through the holder (22) and extends toward an upper direction of the insulator (14). The insulator (14) includes "protrusion accommodation groove (211) of the insulator (14)" which is a protrusion accommodation groove (for example, recessed shape) for accommodating the protrusion. When the protrusion is accommodated into the accommodation groove, the strength of the microwave reception antenna can be maintained by not forming a hole shape in the microwave reception antenna.

The medical treatment instrument (200) according to the present invention fixes the microwave reception antenna (10) and the insulator (14) by connecting two protrusions each formed in a different direction and the accommodation groove for the two protrusions to each other. Accordingly, a come-off prevention of the two antennas can be achieved while maintaining the strength of the two antennas (in particular, the microwave application antenna (12)).

Although the material of the protrusion (pin) is not particularly limited, stainless steel (SUS 304) can be exemplified.

### (Opening and Closing Operation of Microwave Reception Antenna (10) in Medical Treatment Instrument (200))

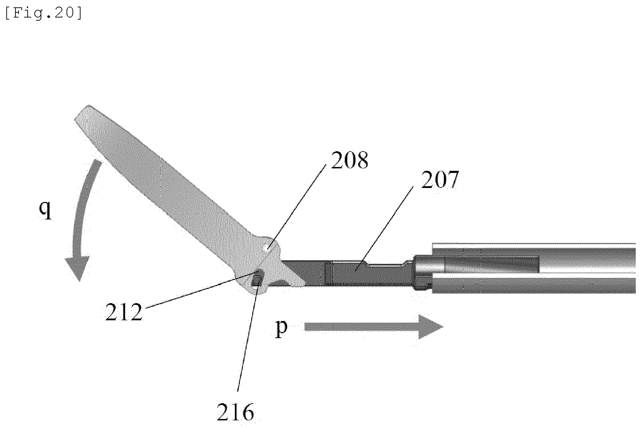

The medical treatment instrument (200) according to the present invention includes, as illustrated in FIG. 20, the traction rod (207) (material thereof is, for example, a metal) including a pin (216) that extends in an inner direction of the treatment instrument at a distal end of traction rod (207) and "microwave reception antenna peak-side terminal end hole (212)" which is a hole at the peak-side terminal end of the microwave reception antenna (10). The pin is inserted into the hole.

As illustrated in FIG. 20, when the traction rod (207) is moved toward a terminal end direction of the treatment instrument (see arrow "p"), the pin (216) of the traction rod (207) is similarly moved toward the terminal end direction (see arrow "p"), and the movement is changed to a rotation operation of the microwave reception antenna (10) (see arrow "q") with the protrusion (208) at the terminal end of the microwave reception antenna serving as a fulcrum. Specifically, the traction rod (207) is moved toward the terminal end direction of the treatment instrument to close the microwave reception antenna (10), and the rod is moved toward the extension direction of the treatment instrument to open the microwave reception antenna (10).

The medical treatment instrument (200) according to the present invention adopts an opening and closing operation mechanism of the microwave reception antenna (10) described above, to thereby achieve downsizing of the entire holder cover (19). The downsizing of the entire holder cover (19) allows the distal end of the medical treatment instrument (1) to be thinner, to thereby make insertion of the distal end of the treatment instrument into fine tissue or a tumor easier.

### (360° Rotation of Antennas in Medical Treatment Instrument (200))

The medical treatment instrument (200) according to the present invention may include the rotator (200), the shaft (201) that penetrates inside of the rotator, the main body cover (213), and a semi-rigid cable rotation connector (218) and a coaxial cable rotation connector (217) that are accommodated in the main body cover.

The semi-rigid cable (206) and the coaxial cable (217) are each connected to each other through intermediation of the semi-rigid cable rotation connector (218) and the coaxial cable rotation connector (217).

With both connectors, through rotation of the rotator (202), the shaft (201) is rotated 360°, and the antennas are also allowed to be rotated 360°.

### (Switch Cover (215) of Medical Treatment Instrument (200))

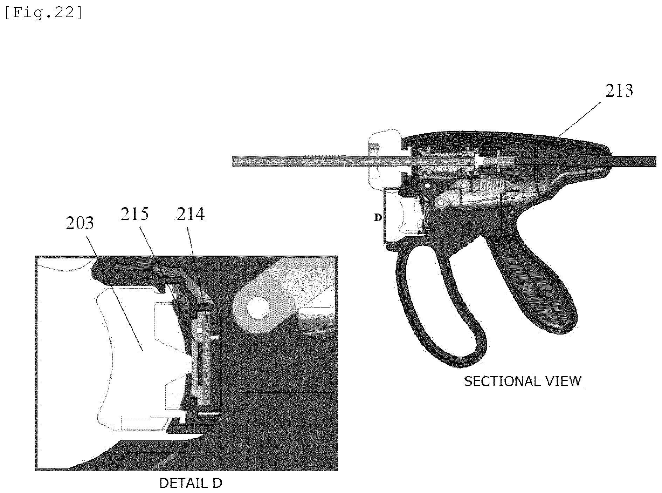

The medical treatment instrument (200) according to the present invention may include, as illustrated in FIG. 22, the hand switch (203), the main body cover (213), a microwave radiation substrate (214) that receives power of the hand switch accommodated in the main body cover, and a switch cover (215) accommodated in the main body cover. Although the material of the switch cover (215) is not particularly limited, elastomer and the like can be exemplified. In addition, the switch cover (215) is located between the hand switch (203) and the microwave radiation substrate (214). The hand switch (203) is not directly in contact with the microwave radiation substrate (214). The switch cover (215) exerts a water-resistant effect and an antistatic effect for the microwave radiation substrate (214) and provides an aging degradation prevention for the microwave radiation substrate (214).

### (Structure of Holder Cover (19) of Medical Treatment Instrument (200))

A shape on the terminal end side of the holder cover (19) of the medical treatment instrument (200) according to the present invention may be, as required, as illustrated in FIG. 17, a tapered shape (219). The tapered shape can prevent the holder cover (19) from coming off in the extension direction. Further, the holder cover (19) can easily be removed from the terminal end direction.

Further, the shaft (201) may include, as illustrated in FIG. 10, one or more or a plurality of claw-shaped portions (220). In addition, the holder cover (19) may include, as illustrated in FIG. 17, one or more or a plurality of through holes (221) that accommodate the claw-shaped portions. When the claw-shaped portions are inserted into the through holes, falling off of the holder cover (19) can be prevented.

### Example 1

### (Use of Medical Treatment Instrument (100))

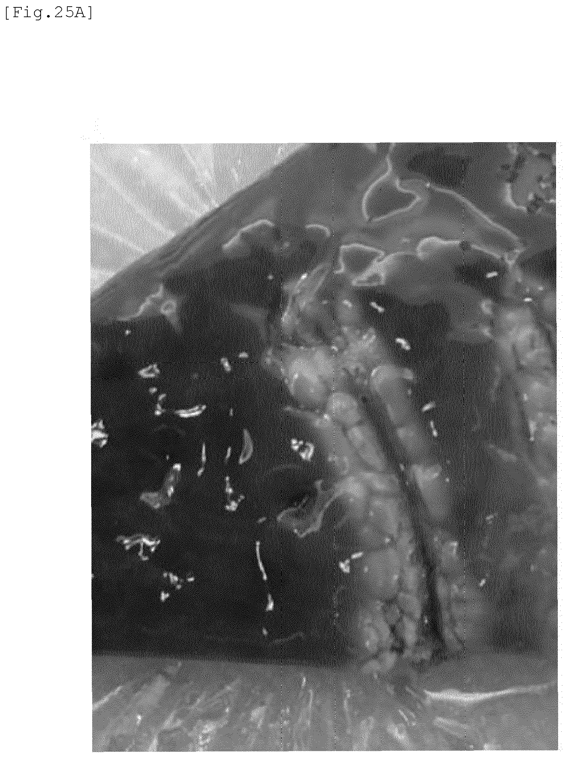

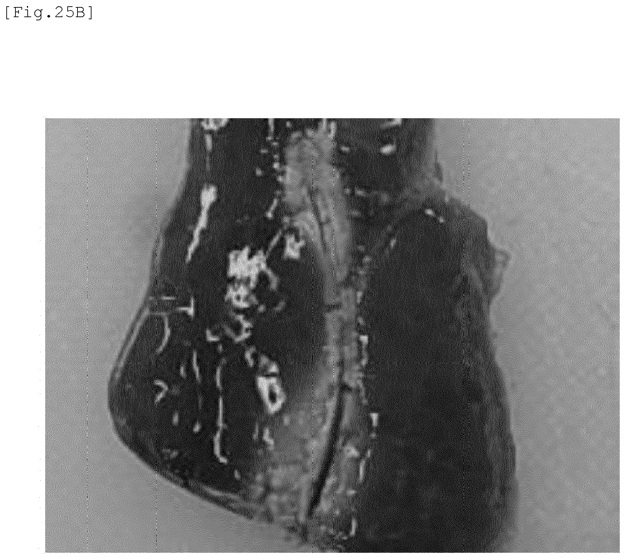

The inventors of the present invention used the medical treatment instrument (100) illustrated in FIG. 1 to coagulate porcine liver tissue and checked a coagulation surface. When the medical treatment instrument (100) illustrated in FIG. 1 was used, as illustrated in FIG. 25A, it was confirmed that even coagulation was performed. On the other hand, when the related-art medical treatment instrument was used, as illustrated in FIG. 25B, it was confirmed that there was a part that was not fully coagulated in a center of the coagulate and this led to confirm uneven coagulation.

Further, occurrence of sparks was not observed.

### Example 2

### (Use of Medical Treatment Instrument (200))

It was confirmed that, with use of the medical treatment instrument (200) illustrated in FIG. 2, similarly to the medical treatment instrument (100) illustrated in FIG. 1, even coagulation could be performed.

Further, occurrence of sparks was not observed.

### Reference Signs List

1 medical treatment tool
10 microwave reception antenna
11 first electrode
12 microwave application antenna
13 second electrode
14 insulator
17 coaxial cable
18 microwave transmission coaxial cable
19 holder cover
20 inner protrusion of microwave application antenna
21 connector
22 holder
100 medical treatment instrument including handle
101 straight portion of antenna cover
102 handle 1 (application)
103 handle 2 (reception)
104 grip 1 (application)
105 grip 2 (reception)
106 finger hook 1 (application)
107 finger hook 2 (reception)
108 ring 1(application)
109 ring 2(reception)
110 coaxial cable protection member
111 distal end shape
112 main support plate
113 auxiliary support plate
114 antenna cover
200 medical treatment instrument including lever
201 shaft
202 rotator
203 hand switch
204 lever
205 grip portion
206 semi-rigid cable
207 traction rod including pin
208 protrusion that extends toward inner surface of insulator
209 protrusion accommodation groove located on inner side of insulator
210 protrusion that extends toward insulator
211 protrusion accommodation groove of insulator
212 microwave reception antenna peak-side terminal end hole
213 main body cover
214 microwave radiation substrate
215 switch cover
216 pin
217 coaxial cable rotation connector
218 semi-rigid cable rotation connector
219 tapered shape
220 claw-shaped portion
221 through hole

## Claims

1. A medical treatment instrument (1), comprising:
a first electrode (11) including a microwave reception antenna (10);
a second electrode (13) including a microwave application antenna (12) with an insulator (14), the insulator (14) being installed on a surface opposed to the microwave reception antenna (10);
a coaxial cable (17) or a semi-rigid cable (206) including a center conductor and an outer conductor, a distal end of the center conductor and a distal end of the outer conductor each being directly or indirectly connected to a terminal end of the microwave application antenna (12) and a terminal end of the microwave reception antenna (10);
a holder cover (19), the holder cover (19) accommodating a part of the microwave application antenna (12) and a part of the microwave reception antenna (10) on a terminal end side thereof;
a holder (22) accommodated in the holder cover (19); and
an inner protrusion (20) of the microwave application antenna (12), the inner protrusion (20) extending toward an inner side of the holder (22).

2. The medical treatment instrument according to claim 1, wherein the inner protrusion of the microwave application antenna is configured to be brought into contact with the inner side of the holder when load is applied to an outer surface of the microwave application antenna facing away from the microwave reception antenna

3. The medical treatment instrument according to claim 1, further comprising an antenna cover (114), wherein the antenna cover (114) accommodates a connection portion between the microwave application antenna (12) and the coaxial cable (17), or the semi-rigid cable (206).

4. The medical treatment instrument according to claim 3, wherein the antenna cover (114) includes a straight portion (101) that accommodates a connection portion between the microwave application antenna (12) and the coaxial cable (17).

5. The medical treatment instrument according to claim 1,
wherein the medical treatment instrument includes a first handle (102), a second handle (103), a first arm (104), a second arm (105), a first finger hook (106), a second finger hook (107), a first ring (108), and a second ring (109) in order to form forceps, and
wherein the first finger hook (106) and the first ring (108) are each located in a substantially symmetrical manner with the second finger hook (107) and the second ring (109) with respect to a center line of the treatment instrument.

6. The medical treatment instrument according to claim 5, wherein the holder cover (19) includes a coaxial cable protection member (110) on a terminal end side of the holder cover (19), and wherein the coaxial cable protection member (110) is installed between the first handle (102) and the second handle (103), and the coaxial cable (17).

7. The medical treatment instrument according to claim 5 or 6, wherein the medical treatment instrument includes a main support plate (112) and an auxiliary support plate (113), and wherein the main support plate (112) and the auxiliary support plate (113) are connected to a terminal end of the microwave reception antenna (10), and the terminal end of the microwave reception antenna (10) and a distal end of the auxiliary support plate (113) are connected to each other with a distal end of the main support plate (112) sandwiched therebetween.

8. The medical treatment instrument according to claim 5, wherein the second handle (103) has a distal end shape (111) that prevents tissue from entering a gap defined between the holder cover (19) and the second handle (103).

9. The medical treatment instrument according to claim 1 or 2,
wherein a distal end of the semi-rigid cable (206) has a protruding shape, and the terminal end of the microwave application antenna (12) has a connection groove that is a slit formed in a recessed manner, and
wherein the protruding shape and the connection groove form a connection structure.

10. The medical treatment instrument according to claim 1 or 2,
wherein the medical treatment instrument includes a traction rod (207), and the traction rod (207) includes a pin (216) at an end of the rod (207), and
wherein the microwave reception antenna (10) includes a hole (212) at a terminal end of the antenna (10), and the pin (216) is inserted into the hole (212).

11. The medical treatment instrument according to claim 1 or 2, wherein the medical treatment instrument includes a main body cover (213) including any one or more of a shaft (201), a rotator (202), a hand switch (203), a lever (204), and a grip portion (205).

12. The medical treatment instrument according to claim 11,
wherein the medical treatment instrument includes a hand switch (203), the main body cover (213), a microwave radiation substrate (214) that receives power of the hand switch (203) accommodated in the main body cover (213), and a switch cover (215) accommodated in the main body cover (213), and
wherein the switch cover (215) is located between the hand switch (203) and the microwave radiation substrate (214).

13. The medical treatment instrument according to claim 11,
wherein the shaft (201) includes one or more or a plurality of claw-shaped portions (220), and the holder cover (19) includes one or more or a plurality of through holes (221) that accommodate the claw-shaped portions (220), and
wherein the claw-shaped portions (220) are inserted into the through holes (221).

## Patentansprüche

1. Medizinische Behandlungsvorrichtung (1), aufweisend:
eine erste Elektrode (11), die eine Mikrowellenempfangsantenne (10) umfasst;
eine zweite Elektrode (13), die eine Mikrowellenanwendungsantenne (12) mit einem Isolator (14) umfasst, wobei der Isolator (14) auf einer Oberfläche gegenüber der Mikrowellenempfangsantenne (10) installiert ist;
ein Koaxialkabel (17) oder ein halbstarres Kabel (206), das einen Mittelleiter und einen Außenleiter umfasst, ein distales Ende des Mittelleiters und ein distales Ende des Außenleiters jeweils direkt oder indirekt mit einem Anschlussende der Mikrowellenanwendungsantenne (12) und einem Anschlussende der Mikrowellenempfangsantenne (10) verbunden sind;
eine Halterabdeckung (19), die Halterabdeckung (19) einen Teil der Mikrowellenanwendungsantenne (12) und einen Teil der Mikrowellenempfangsantenne (10) auf einer Anschlussendeseite davon aufnimmt;
einen Halter (22), der in der Halterabdeckung (19) aufgenommen ist; und
einen inneren Vorsprung (20) der Mikrowellenanwendungsantenne (12), wobei sich der innere Vorsprung (20) in Richtung einer Innenseite des Halters (22) erstreckt.

2. Medizinische Behandlungsvorrichtung gemäß Anspruch 1, wobei der innere Vorsprung der Mikrowellenanwendungsantenne konfiguriert ist, mit der Innenseite des Halters in Kontakt gebracht zu werden, wenn eine Last auf eine äußere Oberfläche der Mikrowellenanwendungsantenne aufgebracht wird, die von der Mikrowellenempfangsantenne abgewandt ist.

3. Medizinische Behandlungsvorrichtung gemäß Anspruch 1, ferner aufweisend eine Antennenabdeckung (114), wobei die Antennenabdeckung (114) einen Verbindungsabschnitt zwischen der Mikrowellenanwendungsantenne (12) und dem Koaxialkabel (17) oder dem halbstarren Kabel (206) aufnimmt.

4. Medizinische Behandlungsvorrichtung gemäß Anspruch 3, wobei die Antennenabdeckung (114) einen geraden Abschnitt (101) umfasst, der einen Verbindungsabschnitt zwischen der Mikrowellenanwendungsantenne (12) und dem Koaxialkabel (17) aufnimmt.

5. Medizinische Behandlungsvorrichtung gemäß Anspruch 1,
wobei die medizinische Behandlungsvorrichtung einen ersten Griff (102), einen zweiten Griff (103), einen ersten Arm (104), einen zweiten Arm (105), einen ersten Fingerhaken (106), einen zweiten Fingerhaken (107), einen ersten Ring (108) und einen zweiten Ring (109) umfasst, um eine Zange zu bilden, und
wobei der erste Fingerhaken (106) und der erste Ring (108) jeweils in einer im Wesentlichen symmetrischen Weise mit dem zweiten Fingerhaken (107) und dem zweiten Ring (109) in Bezug auf eine Mittellinie der Behandlungsvorrichtung platziert sind.

6. Medizinische Behandlungsvorrichtung gemäß Anspruch 5, wobei die Halterabdeckung (19) ein Koaxialkabelschutzelement (110) an einer Anschlussendeseite der Halterabdeckung (19) umfasst, und wobei das Koaxialkabelschutzelement (110) zwischen dem ersten Griff (102) und dem zweiten Griff (103) und dem Koaxialkabel (17) installiert ist.

7. Medizinische Behandlungsvorrichtung gemäß Anspruch 5 oder 6, wobei die medizinische Behandlungsvorrichtung eine Hauptträgerplatte (112) und eine Hilfsträgerplatte (113) umfasst, und wobei die Hauptträgerplatte (112) und die Hilfsträgerplatte (113) mit einem Anschlussende der Mikrowellenempfangsantenne (10) verbunden sind und das Anschlussende der Mikrowellenempfangsantenne (10) und ein distales Ende der Hilfsträgerplatte (113) miteinander verbunden sind, mit einem dazwischenliegenden distalen Ende der Hauptträgerplatte (112).

8. Medizinische Behandlungsvorrichtung gemäß Anspruch 5, wobei der zweite Griff (103) eine distale Endform (111) aufweist, die verhindert, dass Gewebe in einen Spalt eintritt, der zwischen der Halterabdeckung (19) und dem zweiten Griff (103) definiert ist.

9. Medizinische Behandlungsvorrichtung gemäß Anspruch 1 oder 2,
wobei ein distales Ende des halbstarren Kabels (206) eine vorstehende Form hat und das Anschlussende der Mikrowellenanwendungsantenne (12) eine Verbindungsnut hat, die ein Schlitz ist, der auf eine vertiefte Weise ausgebildet ist, und
wobei die vorstehende Form und die Verbindungsnut eine Verbindungsstruktur bilden.

10. Medizinische Behandlungsvorrichtung gemäß Anspruch 1 oder 2,
wobei die medizinische Behandlungsvorrichtung eine Zugstange (207) umfasst und die Zugstange (207) einen Stift (216) an einem Ende der Stange (207) umfasst, und
wobei die Mikrowellenempfangsantenne (10) ein Loch (212) an einem Anschlussende der Antenne (10) umfasst und der Stift (216) in das Loch (212) eingeführt ist.

11. Medizinische Behandlungsvorrichtung gemäß Anspruch 1 oder 2, wobei die medizinische Behandlungsvorrichtung eine Hauptkörperabdeckung (213) umfasst, die eines oder mehrere von einem Schaft (201), einem Rotator (202), einem Handschalter (203), einem Hebel (204) und einem Griffabschnitt (205) umfasst.

12. Medizinische Behandlungsvorrichtung gemäß Anspruch 11,
wobei die medizinische Behandlungsvorrichtung einen Handschalter (203), die Hauptkörperabdeckung (213), ein Mikrowellenstrahlungssubstrat (214), das die Leistung des in der Hauptkörperabdeckung (213) aufgenommenen Handschalters (203) aufnimmt, und eine Schalterabdeckung (215) umfasst, die in der Hauptkörperabdeckung (213) aufgenommen ist, und
wobei sich die Schalterabdeckung (215) zwischen dem Handschalter (203) und dem Mikrowellenstrahlungssubstrat (214) befindet.

13. Medizinische Behandlungsvorrichtung gemäß Anspruch 11,
wobei der Schaft (201) einen oder mehrere oder eine Mehrzahl klauenförmiger Abschnitte (220) umfasst und die Halterabdeckung (19) ein oder mehrere oder eine Mehrzahl von Durchgangslöchern (221) umfasst, die die klauenförmigen Abschnitte (220) aufnehmen, und
wobei die klauenförmigen Abschnitte (220) in die Durchgangslöcher (221) einführt sind.

## Revendications

1. Instrument de traitement médical (1), comprenant :
une première électrode (11) comprenant une antenne de réception de micro-ondes (10) ;
une seconde électrode (13) comprenant une antenne d'application de micro-ondes (12) avec un isolant (14), l'isolant (14) étant installé sur une surface opposée à l'antenne de réception de micro-ondes (10) ;
un câble coaxial (17) ou un câble semi-rigide (206) comprenant un conducteur central et un conducteur externe, une extrémité distale du conducteur central et une extrémité distale du conducteur externe étant chacune directement ou indirectement raccordée à une extrémité terminale de l'antenne d'application de micro-ondes (12) et à une extrémité terminale de l'antenne de réception de micro-ondes (10) ;
un couvercle de support (19), le couvercle de support (19) recevant une partie de l'antenne d'application de micro-ondes (12) et une partie de l'antenne de réception de micro-ondes (10) sur un côté d'extrémité terminale de celui-ci ;
un support (22) reçu dans le couvercle de support (19) ; et
une saillie interne (20) de l'antenne d'application de micro-ondes (12), la saillie interne (20) s'étendant vers un côté interne du support (22).

2. Instrument de traitement médical selon la revendication 1, ladite saillie interne de l'antenne d'application de micro-ondes étant conçue pour être mise en contact avec le côté interne du support lorsqu'une charge est appliquée sur une surface externe de l'antenne d'application de micro-ondes opposée à l'antenne de réception de micro-ondes.

3. Instrument de traitement médical selon la revendication 1, comprenant en outre un couvercle d'antenne (114), ledit couvercle d'antenne (114) recevant une partie de raccordement entre l'antenne d'application de micro-ondes (12) et le câble coaxial (17), ou le câble semi-rigide (206).

4. Instrument de traitement médical selon la revendication 3, ledit couvercle d'antenne (114) comprenant une partie droite (101) qui reçoit une partie de raccordement entre l'antenne d'application de micro-ondes (12) et le câble coaxial (17).

5. Instrument de traitement médical selon la revendication 1,
ledit instrument de traitement médical comprenant une première poignée (102), une seconde poignée (103), un premier bras (104), un second bras (105), un premier crochet à doigt (106), un second crochet à doigt (107), un premier anneau (108) et un second anneau (109) afin de former des pinces, et
ledit premier crochet à doigt (106) et ledit premier anneau (108) étant chacun situés de manière sensiblement symétrique avec le second crochet à doigt (107) et le second anneau (109) par rapport à une ligne centrale de l'instrument de traitement.

6. Instrument de traitement médical selon la revendication 5, ledit couvercle de support (19) comprenant un élément de protection de câble coaxial (110) sur un côté d'extrémité terminale du couvercle de support (19), ledit élément de protection de câble coaxial (110) étant installé entre la première poignée (102) et la seconde poignée (103), et le câble coaxial (17).

7. Instrument de traitement médical selon la revendication 5 ou 6, ledit instrument de traitement médical comprenant une plaque de support principale (112) et une plaque de support auxiliaire (113), et ladite plaque de support principale (112) et ladite plaque de support auxiliaire (113) étant raccordées à une extrémité terminale de l'antenne de réception de micro-ondes (10), et ladite extrémité terminale de l'antenne de réception de micro-ondes (10) et une extrémité distale de la plaque de support auxiliaire (113) étant raccordées l'une à l'autre avec une extrémité distale de la plaque de support principale (112) prise en sandwich entre elles.

8. Instrument de traitement médical selon la revendication 5, ladite seconde poignée (103) présentant une forme d'extrémité distale (111) qui empêche le tissu de pénétrer dans un espace défini entre le couvercle de support (19) et la seconde poignée (103).

9. Instrument de traitement médical selon la revendication 1 ou 2,
une extrémité distale du câble semi-rigide (206) présentant une forme saillante, et ladite extrémité terminale de l'antenne d'application de micro-ondes (12) comportant une rainure de raccordement qui est une fente formée de manière évidée, et
ladite forme saillante et ladite rainure de raccordement formant une structure de raccordement.

10. Instrument de traitement médical selon la revendication 1 ou 2,
ledit instrument de traitement médical comprenant une tige de traction (207), et ladite tige de traction (207) comprenant une broche (216) au niveau d'une extrémité de la tige (207), et
ladite antenne de réception de micro-ondes (10) comprenant un trou (212) au niveau d'une extrémité terminale de l'antenne (10), et ladite broche (216) étant insérée dans le trou (212).

11. Instrument de traitement médical selon la revendication 1 ou 2, ledit instrument de traitement médical comprenant un couvercle de corps principal (213) comprenant un ou plusieurs éléments parmi un arbre (201), un rotateur (202), un commutateur manuel (203), un levier (204) et une partie de préhension (205).

12. Instrument de traitement médical selon la revendication 11,
ledit instrument de traitement médical comprenant un commutateur manuel (203), le couvercle de corps principal (213), un substrat de rayonnement de micro-ondes (214) qui reçoit l'alimentation du commutateur manuel (203) logé dans le couvercle de corps principal (213), et un couvercle de commutateur (215) logé dans le couvercle de corps principal (213), et
ledit couvercle de commutateur (215) étant situé entre le commutateur manuel (203) et le substrat de rayonnement de micro-ondes (214).

13. Instrument de traitement médical selon la revendication 11,
ledit arbre (201) comprenant une ou plusieurs ou une pluralité de parties en forme de griffe (220), et ledit couvercle de support (19) comprenant un ou plusieurs ou une pluralité de trous traversants (221) qui reçoivent les parties en forme de griffe (220), et
lesdites parties en forme de griffe (220) étant insérées dans les trous traversants (221).
